(19) Europäisches Patentamt
European Patent Office
Office européen des brevets

(11) **EP 2 168 473 A1**

(12) **EUROPEAN PATENT APPLICATION**
published in accordance with Art. 153(4) EPC

(43) Date of publication:
**31.03.2010 Bulletin 2010/13**

(51) Int Cl.:
***A61B 3/032*** (2006.01)

(21) Application number: **08787620.7**

(22) Date of filing: **20.06.2008**

(86) International application number:
**PCT/ES2008/000439**

(87) International publication number:
**WO 2009/007477 (15.01.2009 Gazette 2009/03)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **04.07.2007 ES 200701891**

(71) Applicant: **Universidad De Murcia**
**30003 Murcia (ES)**

(72) Inventors:
• **ARTAL SORIANO, Pablo**
 **E-30003 Murcia (ES)**
• **MANZANERA ROMAN, Silvestre**
 **E-30003 Murcia (ES)**

(74) Representative: **Temino Ceniceros, Ignacio et al**
**Abril Abogados, S.L.**
**C/ Amador de los Rios, 1-1°**
**28010 Madrid (ES)**

(54) **AUTOMATED METHOD FOR MEASURING READING ACUITY**

(57)    The procedure is based on the use of a reading test, whose fundamental objective is to remove the traditional Examiner from the process; in a way that not only removes the inherent costs of having an Examiner present but also eliminates the risk of errors being made by the Examiner.

For this, the test introduces computer controlled adjustment and/or forced selection methods, which enables to achieve precise results without the aid of an Examiner. Additionally, the words are displayed using IT systems with a variety of supporting units such as a monitor or TV screen, a display, etc.

FIG. 1

**Description**

## PURPOSE OF THE INVENTION

This invention refers to a new procedure for measuring the general vision quality; and in particular, reading acuteness in a variety of situations and with the subject or patient using any ophthalmic device such as lenses, intraocular lenses or contact lenses.

Thus, the invention is categorized in the fields of ophthalmology, optic vision and optometry.

## INVENTION BACKGROUND

In the clinical field for detecting ocular disease or visual defects as well as in the investigation field for testing new ophthalmic devices, the use of procedures or tests that enable the vision quality of the patient to be measured are required. Traditional tests measure visual acuteness (VA) or sensitivity to contrast (SC), normally through the use of laminates or static cards and in some cases through the use of IT systems. VA tests try to determine the size of the smallest item the patient is able to see, while the SC tests determines the minimum contrast at which the patient is able to differentiate regions of a determined spatial frequency. Both tests provide valuable information regarding visual quality but they both have limitations. Therefore, other newer procedures are needed that enable specifically to measure the capabilities of the patient for carrying out common, every day visual tasks.

Among these tasks, one of the most important ones is reading. At first, the reading tests were developed for studying the importance of the different variables that play a part in the reading process, (Legge G.E. D.G. Pelli, G.S. Rubin & M.M. Schleske. Psychophysics of reading. I. Normal vision. Vision Research, 25, 239-252, 1985) and for measuring the visual capabilities of patients with low vision (Legge G.E., G.S. Rubin, D.G. Pelli & M.M. Schleske. Psychophysics of reading. II. Low vision. Vision Research, 25, 253-266, 1985). These tests involve showing the patient unrelated phrases or words he must read out loud and as fast as possible while the examiner records the errors made. A measurement of the reading speed is obtained from the number of errors and the number of words read per minute. Numerous studies have been carried out on this subject, primarily at the Minnesota Laboratory for Low-Vision Research, where the different parameters that may affect reading, such as contrast have been investigated (Legge G.E., G.S. Rubin & A. Luebker. Psychophysics of reading. V. The role of contrast in normal vision. Vision Research, 27, 1165-1171, 1987), illumination wavelength (Legge G.E. & G.S. Rubin. Psychophysics of reading. IV. Wavelength effects in normal and low vision. Journal of the Optical Society of America, A3, 40-51, 1986), or the size of the font or letters (Chung S.T.L., J.S. Mansfield & G.E. Legge. Psychophysics of reading. XVIII. The effect of print size on reading speed in normal peripheral vision. Vision Research, 38, 2949-2962, 1998). Using one of these tests as a base, the one called Minnesota Low-Vision Reading Test, abbreviated as Mnread (Legge G.E., J.A. Ross, A. Luebker & J.M. LaMay. Psychophysics of reading. VIII. The Minnesota low-vision reading test. Optometry and Vision Science, 66, 843-853, 1989), a set of rules or directives that must be followed while conducting reading tests were developed, which are listed in the MNread 2000 project (http://gandalf.psych.umn.edu/gellab/ MNREAD/). Apart from the tests that measure reading speed, this project also lists other tests that measure reading acuteness and the critical letter size (minimum letter size that can be read at maximum speed).

Until now, when the visual capability of a person is evaluated using a traditional reading test, the procedure consists in the Examiner counting the number of errors made by the patient while the patient is reading a series of phrases. This requires the Examiner's full attention and may lead to miscounting of the reading errors, which would consequently alter the test results. Additionally, the usage of large or complicated texts may not be adequate in different clinical or experimental situations.

Therefore, the standard procedure to date for evaluating the visual capability of a patient using a reading test is based on counting the errors made by the patient while he is reading a specific set of phrases. This procedure of counting errors is carried out manually by the Examiner, which requires all of their time and their full attention and may result in miscounting.

## DESCRIPTION OF THE INVENTION

The procedure of this invention proposes to fully and satisfactorily resolve the problem mentioned above.

In a more specific way and in order to prevent the two above mentioned problems, this invention proposes the use of computer assisted adjustment and/or forced selection applied to the reading test. In the adjustment method, the patient must adjust or modify some of the different parameters that affect the reading (letter size, contrast, etc...) subjectively trying to find the value of said parameter below which he cannot read properly. In the generally described forced selection method, the patient is shown words or phrases, or group of these, which he must try to read; and afterwards, provide a response (choosing between different options) to a previously asked question. A measurement of the patient's reading capability is obtained from the correct and erroneous answers provided by him.

## DESCRIPTION OF THE DRAWINGS

To complement the description that is being carried out and for the purpose of better understanding the characteristics of the invention, in accordance with a preferred example for carrying it out in a practical way, a set of drawings are provided along with their description, which represent the following in an illustrative but not a limiting fashion:

Figure 1. - Shows a basic general schematic of the computer assisted automatic procedure for carrying out the reading test: The patient reads the word or phrase that is displayed on the computer screen and then sends information to the computer regarding what he/she has read in a pre-established way.

Figure 2. - Shows the typical psychometric curve of a test that is carried out via the forced selection procedure. This curve represents the percentage of correct answers depending on the value of the parameter, whose threshold wants to be obtained. This threshold corresponds to a percentage of correct answers, which has been previously established according to the number of response options available.

Figure 3. - Via a coordinate system, it also shows the typical behaviour of the parameter value, whose threshold is to be determined during a test that is carried out via the forced selection procedure. The value shown to the patient oscillates around the threshold value, which is being calculated as the performance number increases.

Figure 4. - Shows how the letter size is selected according to the height of the lower case letters.

Figure 5. - Shows a computer screen window that shows the control for the reading test in the adjustment mode.

Figure 6. - Shows a list of real word and false words shown in the forced selection mode of the reading test.

Figure 7. - Shows a reading test control and monitoring window in the forced selection mode.

Figure 8. - Shows a window of the database of words used in the words test.

Figure 9. - Shows another window displayed on the computer screen that shows the configuration of the reading test.

## PREFERRED EMBODIMENT OF THE INVENTION

The procedure proposed by this invention, whose purpose is to free up the Examiner from the arduous task of carrying out the evaluation, with the consequent and complementary elimination of personal errors, consists in a procedure or computer aided adjustment and/or forced selection method that is able to objectively quantify the reading capability without Examiner involvement, leaving them exempt of this task and eliminating the risk for making errors.

In order to resolve these two problems, this invention proposes the application of computer assisted adjustment and/or forced selection method applied to the reading test, which is able to objectively quantify the reading capability without intervention from the Examiner.

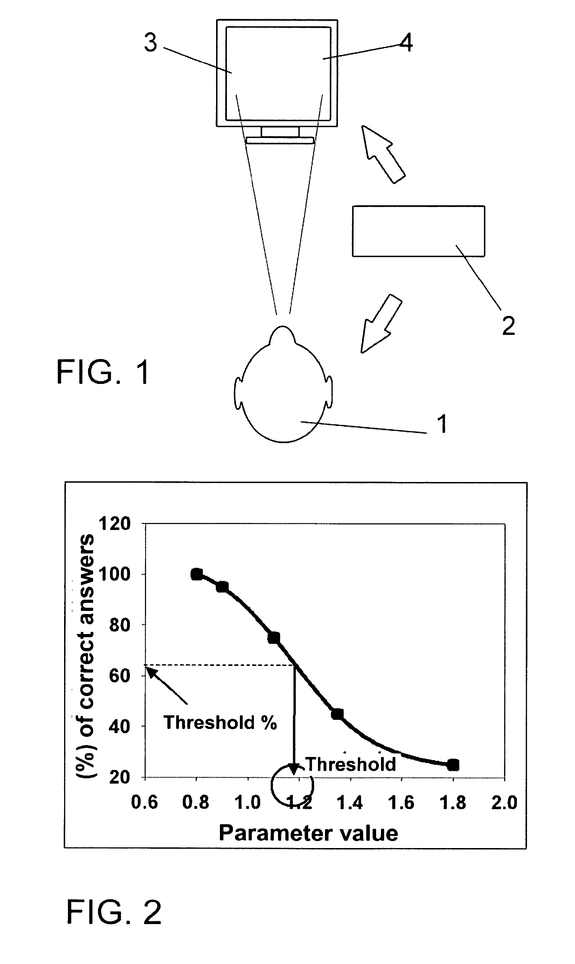

The following can be discerned from the schematic of figure 1, where (1) lists the patient, (2) the computer and (3) the text that appears on the computer screen (4).

Additionally, groups of letters are proposed to be displayed in order to prevent random responses, which in some cases have meaning and in others do not. Only in those cases where the subject can read the word correctly will he or she be able to differentiate the real word from the false one.

Below, we describe the adjustment and forced selection methods of the reading test. In both cases, the patient is shown unrelated words or complete phrases to read. In following and briefly, we will only mention words, while understanding that everything stated also applies to phrases.

In the adjustment method, the computer shows words to the patient, who is able to (by using any computer communication device i.e. keyboard, mouse, etc...) increase or diminish the value of some of the different parameters that affect the reading (letter size, contrast, etc,...) subjectively trying to find the threshold value of said parameter below which he or she cannot read properly.

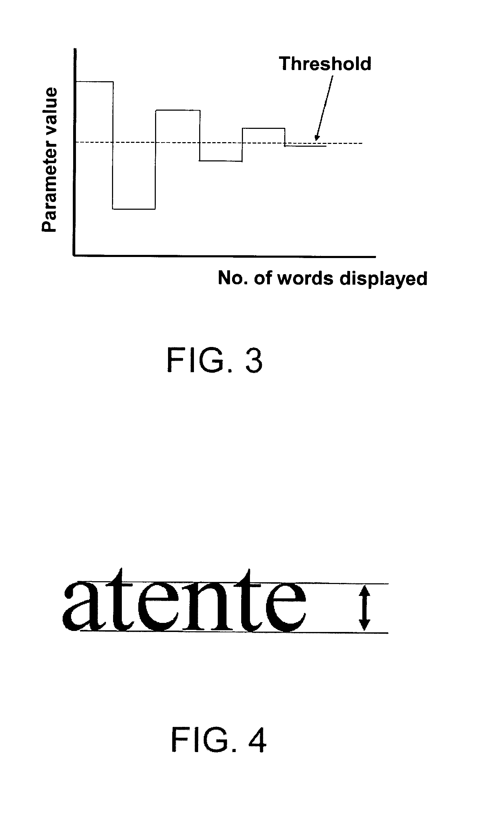

In the forced selection method, the patient is shown a sequence of words or groups of words he must attempt to read. After reading each of these groups, he or she must provide an answer (choosing between several possible options) to a question previously stated at the beginning of the test. The displaying of words and obtaining of responses are computer automated tasks. A measurement of the patient's reading capability is obtained from the correct and erroneous answers provided by the patient, which may be calculated via two procedures. In the first procedure, after obtaining the psychometric curve (percentage of correct answers according to the value of the parameter, whose reading threshold wants to be calculated), a value corresponding to a previously established percentage of correct answers is used as the threshold value (fig. 2). In the second procedure, the value of the parameter of each one of the displayed words is determined by the psychophysical algorithm used (linear staircase, log staircase, Quest...). In these algorithms, the value of the parameter for each new display is provided by the history of the patient's previous responses; in other words, through the patient's correct answers and errors at determined values. After a determined number of displays, the threshold sought has been calculated with enough reliability and is provided as the final test value (fig. 3).

EXAMPLE.-

In the following example, what is described is the procedure for measuring the reading contrast sensitivity (for a determined letter size, contrast threshold that allows reading) and the reading acuteness (minimum size of the letters that make up the word to be read). The words are displayed on the computer monitor that controls the entire process and the subject responds to the test questions via the keyboard by using specific keys.

Like visual acuteness; reading acuteness can be expressed in logMAR, in Snellen fractions, or in decimal notation. Since different size letters exist (for example a, b), the height of a lower case letter is used as shown in (fig. 4)

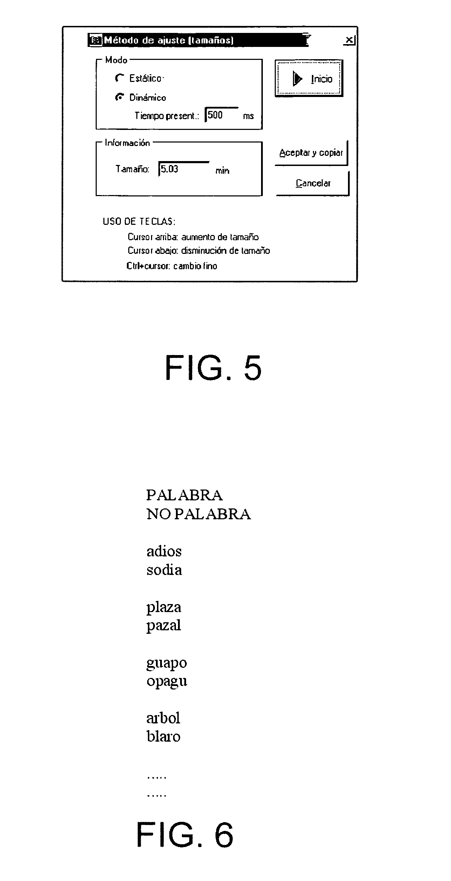

A first implementation of the test is based on the adjustment procedure (fig. 5). The subject is shown words and their task is to change the size or the contrast (depending on what needs to be measured) of the letters using a keyboard until the threshold value is achieved, below which the subject is not able to recognize the displayed word. From this point forward, only sizes will be mentioned, but everything mentioned is also applicable for contrasts. This test can be carried out in a static way, where the word changes only when the subject varies the size, or in a dynamic way, where the words are continuously changing at certain constant time intervals, which are pre-fixed independently from any actions by the subject.

This time is set at 500 ms, which enables the subject to read the word. If this word was always the same, the subject could mistakenly think he or she is really seeing and correctly reading the word, when in reality this may be the effect of the subject remembering said word. In the static mode, changing the size to change the displayed word is required while this is not required in the dynamic mode.

A second method for implementing the test is through the forced selection method. In this case, the subject is shown two words, one after the other separated by a determined time interval. But only one of them is really a word that has meaning since the other word is formed with scrambled letters that make up the first word but without any meaning. We can call it a "non word". Fig. 6 shows some examples.

The subject is asked to identify which of the two words shown is a real word, the first or the second, by pressing the proper key. The number of repetitions required for completing the test and the letter size of each new word displayed is controlled by an adaptive psychophysical QUEST algorithm. Fig. 6 shows a QUEST control window and the monitoring of the entire procedure.

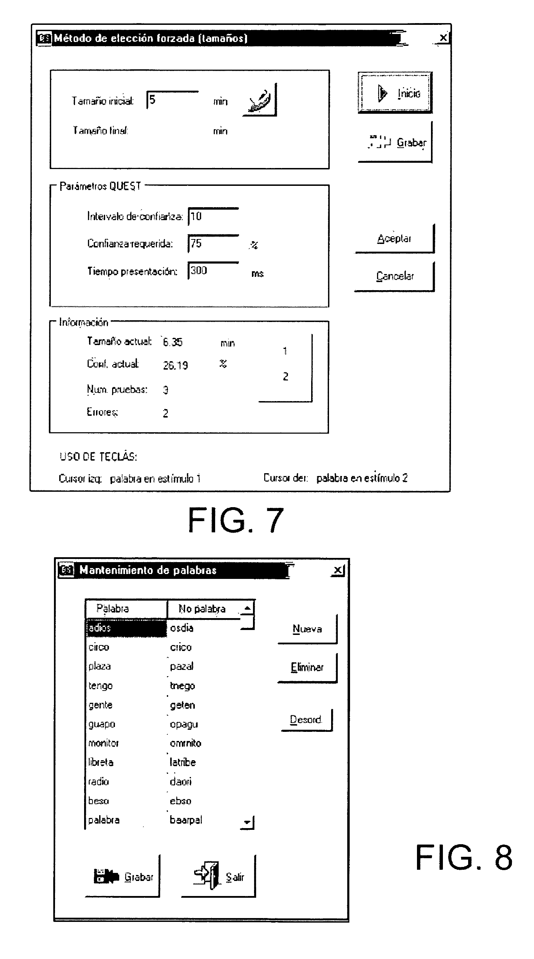

The time that each stimulus (word or non word) is shown to the subject is 500 ms, which is the most adequate to give enough time to read the word and at the same time reduces the total test duration to a minimum, which may oscillate between 3 and 4 minutes. The words used must be simple, commonly used; a total of 200 words is enough to prevent the subject from memorizing them in a reasonable number of sessions. This application enables to maintain the word table and also enables to generate the non word from the real word in an almost automatic way. The process is not completely automatic because at the end it must be checked that the non word actually lacks any meaning. This part of the application is shown in fig. 8.

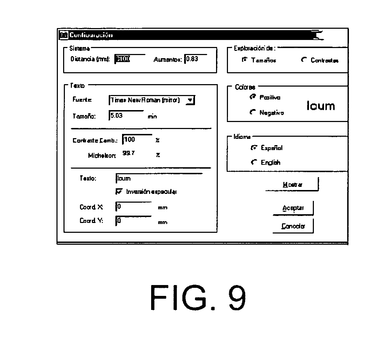

The application incorporates several options required for its use when the subject is not looking directly at the test; instead, he or she is looking through an optical system. This way, it enables to consider the increases, or the existence of a left-right inversion. For this last situation, a special font type is designed in which the design of each letter is identical to the New Times Roman design but each letter is inverted with respect to a vertical axis that passes through its centre. But also required in this case is the existence of an option to invert the order of the letters. Apart from these options, the application incorporates other options such as being able to choose if what is measured by the test is the size threshold or the contrast, if conducted in Spanish or English, or if a light colour letter is displayed over a dark background or vice versa. Fig. 9 shows the configuration window for all these options.

The complete measurement procedure begins with a quick adjustment test. The threshold size value returned by the subject in this test is used as the starting value in the following forced selection test. This way, the total time is reduced by starting with a value that is close to the threshold. Several repeats are carried out and the average value of the results obtained in the series is used as the final value. As in the other test, for quick evaluations, it is possible to average the results of several repetitions using only the adjustment mode. Prior to this, it is necessary to check that the subject's responses are coherent and that their measurements are not excessively dispersed.

Precision in the size with which the different words can be shown is conditioned by the observation distance (D), the system enlargements ( □ ), the pixel size (tp) and by the fact that a whole number of pixels is required for constructing each letter of the word. Depending on the pixels (n) used to display a letter x, the possible reading acuteness (AL) that may be assessed, expressed in decimal notation, are provided by:

$$AL = \frac{\pi}{60 \cdot 180} \frac{D}{n \cdot t_p \cdot \Gamma}$$

The maximum AL is not able to be determined directly by making n=1 because a letter is not able to be represented with only one pixel. Due to the great variety of letter shapes, it is difficult to determine the number of pixels required to be able to reliably represent all of them.

What has been demonstrated is that under normal conditions of test use (D = 3100 mm, $\square\square$= 0.83 and tp = 0.2 mm) the AL of all the evaluated subjects is always below the AL values at which display problems would begin to appear. For example, in a normal subject with a visual acuteness of 1.3, their reading acuteness is 0.18, which requires using (giving n a value in the equation above) 30 pixels, which are more than enough for representing any letter.

**Claims**

1. st.- Automatic procedure for measuring the visual reading acuteness, which uses an automatic reading test **characterized** for using a computer assisted adjustment or forced selection mode that may be carried out without the aid of an Examiner.

2. nd.- Automatic procedure for measuring the visual reading acuteness in accordance with claim 1, **characterized in that** in the adjustment and/or forced selection mode, the computer centralizes and automates the entire process, collecting the patient's responses, which are input by the patient using any peripheral such as a keyboard, mouse or other, and displaying the appropriate word at each moment during the test.

3. rd.- Automatic procedure for measuring the visual reading acuteness in accordance with the claims above, **characterized in that** the method for assessing the reading capability is organized according to several factors such as letter size, contrast and other similar factors.

4. th.- Automatic procedure for measuring the visual reading acuteness in accordance with the claims 1 and 2, **characterized in that** the method for assessing the reading capability is carried out using words, with and without meaning, in an automatic way, which may potentially be applied to the optometry, ophthalmic and similar fields.

FIG. 1

FIG. 2

FIG. 3

FIG. 4

Método de ajuste (tamaños)

Modo

⊙ Estático
⊙ Dinámico

Tiempo present.: 500 ms

▶ Inicio

Información

Tamaño: 5.03 min

Aceptar y copiar

Cancelar

USO DE TECLAS:

Cursor arriba: aumento de tamaño
Cursor abajo: disminución de tamaño
Ctrl+cursor: cambio fino

# FIG. 5

PALABRA
NO PALABRA

adios
sodia

plaza
pazal

guapo
opagu

arbol
blaro

.....
.....

# FIG. 6

FIG. 7

FIG. 8

# FIG. 9

# INTERNATIONAL SEARCH REPORT

| | International application No. |
|---|---|
| | PCT/ ES 2008/000439 |

**A. CLASSIFICATION OF SUBJECT MATTER**

*A61B 3/032* (2006.01)

According to International Patent Classification (IPC) or to both national classification and IPC

**B. FIELDS SEARCHED**

Minimum documentation searched (classification system followed by classification symbols)
A61B3/02

Documentation searched other than minimum documentation to the extent that such documents are included in the fields searched

Electronic data base consulted during the international search (name of data base and, where practicable, search terms used)

CIBEPAT,EPODOC,WPI,BIOSIS,MEDLINE,NPL

**C. DOCUMENTS CONSIDERED TO BE RELEVANT**

| Category* | Citation of document, with indication, where appropriate, of the relevant passages | Relevant to claim No. |
|---|---|---|
| PX | WO 2008064379 A1 (DEXL et al.) 05.06.2008, page 2, line 15 - page 4, line 3;page 4, line 33 - page 5, line 38; claims 1-14; | 1 - 4 |
| X | US 2006078858 A (VROMAN et al.) 13.04.2006, Paragraphs 1, 17, 18, 24, 29, 30; column 3, lines 21-22; claims 1 - 5, 16 - 27, 39, 40, 51, 52, 57 | 1 - 4 |
| X | WO 0134020 A1 (VOYANT CORPORATION) 17.05.2001, abstract; page 1, lines 4-7;page 2, line 10 - page 4, line 4; | 1 - 4 |
| PA | AT 504635 A (RADNER) 15.06.2008, page 6, claim | 1 - 4 |
| A | US 5568209 A (PRIESTER et al.) 22.10.1996, claims 1-27; | 1 - 4 |
| A | WO 0077760 A1 (CAPLYGIN) 21.12.2000, the whole document. | 1 - 4 |

☐ Further documents are listed in the continuation of Box C. ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 25.November.2008 (25.11.2008) | **28 de Noviembre de 2008 (28/11/2008)** |
| Name and mailing address of the ISA/ O.E.P.M.<br><br>Paseo de la Castellana, 75 28071 Madrid, España.<br>Facsimile No. 34 91 3495304 | Authorized officer<br><br>A. Cardenas Villar<br><br>Telephone No. +34 91 349 53 93 |

Form PCT/ISA/210 (second sheet) (July 2008)

## INTERNATIONAL SEARCH REPORT

Information on patent family members

| | International application No. |
|---|---|
| | PCT/ ES 2008/000439 |

| Patent document cited in the search report | Publication date | Patent family member(s) | Publication date |
|---|---|---|---|
| WO 2008064379 A | 05.06.2008 | AT 9488 U | 15.11.2007 |
| US 2006078858 A | 13.04.2006 | NONE | ------------ |
| WO 0134020 A | 17.05.2001 | AU 3078301 A<br>US 6386707 B | 06.06.2001<br>14.05.2002 |
| AT 504635 A | 15.06.2008 | NONE | ------------ |
| US 5568209 A | 22.10.1996 | WO 9632880 A | 24.10.1996<br>24.10.1996<br>24.10.1996 |
| WO 0077760 A | 21.12.2000 | AU 5055400 A<br>EP 1221153 AB<br>EP 20000934820<br>AU 758513 B<br>AT 321491 T<br>DE 60026992 T<br>US 7211050 B<br>US 2008004544 A | 02.01.2001<br>10.07.2002<br>15.06.2000<br>20.03.2003<br>15.04.2006<br>15.03.2007<br>01.05.2007<br>03.01.2008 |

Form PCT/ISA/210 (patent family annex) (July 2008)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Legge G.E. ; D.G. Pelli ; G.S. Rubin ; M.M. Schleske.** Psychophysics of reading. I. Normal vision. *Vision Research,* 1985, vol. 25, 239-252
- **Legge G.E. ; G.S. Rubin ; D.G. Pelli ; M.M. Schleske.** Psychophysics of reading. II. Low vision. *Vision Research,* 1985, vol. 25, 253-266
- **Legge G.E. ; G.S. Rubin ; A. Luebker.** Psychophysics of reading. V. The role of contrast in normal vision. *Vision Research,* 1987, vol. 27, 1165-1171

- **Legge G.E. ; G.S. Rubin.** Psychophysics of reading. IV. Wavelength effects in normal and low vision. *Journal of the Optical Society of America,* 1986, vol. A3, 40-51
- **Chung S.T.L. ; J.S. Mansfield ; G.E. Legge.** Psychophysics of reading. XVIII. The effect of print size on reading speed in normal peripheral vision. *Vision Research,* 1998, vol. 38, 2949-2962
- **Legge G.E. ; J.A. Ross ; A. Luebker ; J.M. LaMay.** Psychophysics of reading. VIII. The Minnesota low-vision reading test. *Optometry and Vision Science,* 1989, vol. 66, 843-853